(19) Europäisches Patentamt
European Patent Office
Office européen des brevets

(11) **EP 4 768 090 A1**

(12) **EUROPEAN PATENT APPLICATION**

(43) Date of publication:
**01.07.2026 Bulletin 2026/27**

(21) Application number: **25227746.2**

(22) Date of filing: **30.12.2025**

(51) International Patent Classification (IPC):
**A63B 24/00** (2006.01)

(52) Cooperative Patent Classification (CPC):
**A63B 24/00; A63B 24/0021; A63B 24/0062;**
A63B 2024/0025; A63B 2024/0056;
A63B 2024/0065; A63B 2220/40; A63B 2220/806;
A63B 2220/836; A63B 2220/89

(84) Designated Contracting States:
**AL AT BE BG CH CY CZ DE DK EE ES FI FR GB
GR HR HU IE IS IT LI LT LU LV MC ME MK MT NL
NO PL PT RO RS SE SI SK SM TR**
Designated Extension States:
**BA**
Designated Validation States:
**GE KH LA MA MD TN**

(30) Priority: **30.12.2024 US 202463740303 P**

(71) Applicant: **Drive Hockey Analytics Inc
Coquitlam, BC V3B 6S2 (CA)**

(72) Inventors:
• **Akella, Krishant**
  **Qoquitlam, V3B 6S2 (CA)**
• **Johnson, Ron**
  **Qoquitlam, V3B 6S2 (CA)**
• **Dahlstedt, Mike**
  **Qoquitlam, V3B 6S2 (CA)**

(74) Representative: **Münzer, Marc Eric
Aalborg Patent ApS
Algade 38, 3
9000 Aalborg (DK)**

(54) **SYSTEMS, DEVICES, ARTICLES AND METHODS FOR CALCULATING SPORTS PERFORMANCE METRICS USING POSITIONAL DATA**

(57) Embodiments of the present disclosure relate to systems, devices, articles and methods for calculating sports performance metrics using positional data. The system includes a local and a cloud computing system. A plurality of player sensor tags and wireless data transceivers measure the location of players during a tracked event. The location data are processed either in real-time by a local edge computing device or post processed by a cloud computing device using mathematical and sports-physics models. The processed data are used to determine acceleration and g-force which in turn determine performance metrics that can be used to provide tactical insight.

FIG. 7

**Description**

**Technical Field**

The disclosure generally relates to the use of wireless devices for measuring positional data of an athlete to estimate performance-related metrics during competitive scenarios. More particularly, the disclosure relates to detecting and measuring positional data of an athlete along with the analysis of their movements, to evaluate performance in sports such as ice hockey, skating or skiing.

**Background**

Over the past several years, the use of data in sports has risen in popularity as a means to make better evaluation, development and tactical decisions, as well as for purposes such as to enhance fan experience through deeper insights and gaming. As the usefulness of data continues to grow, there is a need to capture greater detailed insights and accuracy. For example, identifying the total acceleration (tangential and centripetal) and curvature of an athlete and using the data to estimate fitness metrics for the athlete(s) such as strength and endurance which can help better evaluate an athlete's skill set and provide a means for that same athlete to develop a better technique with less reliance on expert trainers.

Some systems are available for measuring and correcting athlete performance. For example, specialty training rooms are equipped with devices to measure a player's acceleration and trajectory in a controlled environment (e.g., video, accelerometer-based systems, RFID equipment, etc.). The data may be automatically generated and used by the trainer and athlete to adjust and improve their technique. A major disadvantage of such systems is that their use is confined to a single location in a controlled environment and would not be practical to evaluate every movement across all players during actual live sporting events.

Particularly relevant to tracking positional data are systems that can estimate the position of an athlete during a tracked event. US Patent No. 11,179,600 provides different means of estimating the position of an athlete by combining radio-based and optical-based positioning and by utilizing an Inertial Measurement Unit (IMU) to estimate the acceleration. Although the position of an athlete can be estimated as such, radio-based positioning systems are more prone to interference and are less accurate than for example Ultra-Wide Band (UWB) signals which in turn creates the need to rely on multiple positioning means such as radio frequency signals as well as video.

US Patent publication No. 2024/0123289 A1 and US Patent publication No. 2024/0306943 A1 explains a method of measuring performance of an athlete using position data during a set of movements. Multiple wearable sensor devices can be worn by the athlete to measure the performance metrics of a controlled movement. However, this type of performance measurements is mainly related to evaluating the performance of an athlete repeatedly performing a specific set of movements such as weightlifting movements to measure a set of key metrics. It provides little basis to determine the position and acceleration of an athlete among multiple athletes during a gameplay and measure a set of performance metrics pertaining to a sport involving agility and rapid movement across a tracked space such as a sports arena.

Currently, accelerometers in IMU's can measure linear acceleration. However, the precise location of the athletes during game play cannot be measured. Further, accelerometer data itself does not provide other measurements such as a player's centripetal acceleration, velocity or curvature. Fitness is properly characterized by exertion over a period of time. One cannot get exertion directly from unprocessed accelerometer data. Normally one would use Heart Rate from wearables to measure exertion.

The present disclosure may improve existing systems by capturing positional information from players in the same sporting event and more accurately calculating measurements such as total acceleration and deceleration which may include tangential and/or centripetal acceleration and deceleration. Total acceleration and deceleration can therefore include left acceleration/deceleration, right acceleration/deceleration and linear acceleration/deceleration.

A further aspect of the present disclosure is estimating g-force and several other key metrics using the acceleration data which can be used to evaluate performance/fitness of an athlete. The present disclosure may also measure the lean angle which can be used to estimate muscle strength as well as the quality of equipment.

Having precise location, time, technique and performance data provides deeper, richer insight of the skill the athlete possesses rather than limiting to tactical insights that is collected from current sports tracking data systems.

**Summary**

A system of one or more computers can be configured to perform particular operations or actions by virtue of having software, firmware, hardware, or a combination of them installed on the system that in operation causes or cause the system to perform the actions. One or more computer programs can be configured to perform particular operations or actions by virtue of including instructions that, when executed by data processing apparatus, cause the apparatus to perform the actions. One general aspect includes distributing plural transceivers in a sports area. The distributing also includes connecting a tracking device to the athlete; communicatively coupling the transceivers and tracking device; using multi-lateration at the tracking device to determine a series of location data (xi, yi) of the athlete for a corresponding series of timestamps (ti); applying a kalman filter to refine the series of location data, and calculate a series of velocity data and series of acceleration data for the athlete; applying a sports-physics model to the data to calculate total acceleration at each timestamp; calculating and outputting forces experienced by the athlete based on the total acceleration. Other embodiments of this aspect include corresponding computer systems, apparatus, and computer programs recorded on one or more computer storage devices, each configured to perform the actions of the methods.

Implementations may include one or more of the following features. The method where refining the series of location data further may include interpolating the series of location data to correspond to a standard set of times. The quality metrics includes one or more of: force output, force capacity, active load, endurance, exertion during play and recovery. Calculating total acceleration includes calculating acceleration components, including tangential and centripetal acceleration of the athlete. The method may include calculating skate lean or skate technique of the athlete from the calculated acceleration components. The method may include displaying a trace of the athlete using the location data, overlaid on an image of the sports area, with coding to indicate a magnitude or classification of the acceleration. The sports-physics model is particular to a given sport, selected from one of: car racing, skating, skiing, hockey, and lacrosse. The calculated total accelerations are stored for plural athletes in the sporting event and may include computing comparison statistics for total acceleration between athletes or between teams. The calculated total accelerations are stored for plural sporting events and where improvement statistics are computed for the athlete between sporting events. The method may include classifying acceleration components into left acceleration/deceleration, right acceleration/deceleration and linear acceleration/deceleration. The method may include calculating quality metrics. Said calculations are performed over a window of the timestamps that correspond to tracked events stored in a database for that sporting event. The said calculations are performed in real-time. The said calculations are performed after the sporting event. The system may include displaying a trace of the athlete using the location data, overlaid on an image of the sports area, with coding to indicate a magnitude or classification of the acceleration. The sports-physics model is particular to a given sport, selected from one of: car racing, skating, skiing, hockey, and lacrosse. The calculated accelerations are stored for plural athletes in the sporting event and where accelerations comparison statistics are computed between athletes or between teams. The calculated accelerations are stored for plural sporting events and where improvement statistics are computed for the athlete between sporting events. The system may include classifying acceleration components into left acceleration/deceleration, right acceleration/deceleration and linear acceleration/deceleration. Said calculations are performed over a window of the timestamps that correspond to tracked events stored in a database for that sporting event. The tracking device is an UWB package located at one or more of: the athlete's torso, the athlete's stick, athlete's skate, or within a ball or puck. The computer processor further may include a local system and a cloud computing system. Calculating outputting forces further includes calculating and outputting g-force. Implementations of the described techniques may include hardware, a method or process, or computer software on a computer-accessible medium.

One general aspect includes a method of estimating fitness in a sporting event. The method also includes distributing one or more video cameras in a sports area; connecting a stream of images of an athlete using the one or more video cameras; using image processing to identify the athlete and their location within the images to determine a series of location data (xi, yi) of the athlete within the sports area for a corresponding series of timestamps (ti); applying a kalman filter to refine the series of location data, and calculate a series of velocity data and series of acceleration data for the athlete; applying a sports-physics model to the data to calculate total acceleration at each timestamp; calculating and outputting forces experienced by the athlete based on the total acceleration. Other embodiments of this aspect include corresponding computer systems, apparatus, and computer programs recorded on one or more computer storage devices, each configured to perform the actions of the methods.

One general aspect includes a system for estimating fitness in a sporting event. The system also includes plural transceivers distributed in a sports area; a tracking device connectable to an athlete and communicatively couplable to the plural transceivers; and a computer processor, memory and instructions arranged to: use multi-lateration at the tracking device to determine a series of location data (xi, yi) of the athlete for a corresponding series of timestamps (ti); apply a kalman filter to refine the series of location data, and calculate a series of velocity data and series of acceleration data for the athlete; apply a sports-physics model to the data to calculate total acceleration at each timestamp; and calculate and output forces experienced by the athlete based on the total acceleration. Other embodiments of this aspect include corresponding computer systems, apparatus, and computer programs recorded on one or more computer storage devices, each configured to perform the actions of the methods.

Other embodiments of this aspect include corresponding computer systems, apparatus, and computer programs recorded on one or more computer storage devices, each configured to perform the actions of the methods.

Implementations may include one or more of the following features. The system where the said calculations are performed after the sporting event using the cloud computing system. Implementations of the described techniques may include hardware, a method or process, or computer software on a computer-accessible medium.

**Brief description of the drawings**

Systems, devices, and methods are described in greater detail herein with reference to the following figures in which:

FIG. 1 illustrates a segment of a trajectory of a player in accordance with some embodiments of the invention;

FIG. 2 illustrates a block diagram of the system components in accordance with some embodiments of the invention;

FIG. 3 illustrates a block diagram of the cloud computing system of FIG.2;

FIG. 4 illustrates a block diagram of the edge computing device of FIG.2 in accordance with some embodiments of the invention;

FIG. 5 illustrates a block diagram of the electronic player sensor device in accordance with some embodiments of the invention;

FIG. 6 illustrates a block diagram of the wireless location transceivers in accordance with some embodiments of the invention;

FIG. 7 illustrates a block diagram of the wireless tracking system in accordance with some embodiments of the invention;

FIG. 8 illustrates a flow diagram of the performance measurement method in accordance with some embodiments of the invention;

FIG. 9 illustrates a g-force vs. time graph of a player in accordance with some embodiments of the invention;

FIG. 10 illustrates a plot of the trajectory of the player of FIG.9 overlaid on a playing area;

FIG. 11 illustrates a g-force vs. time graph of another player's left acceleration in accordance with some embodiments of the invention;

FIG. 12 illustrates a g-force vs. time graph of yet another player in accordance with some embodiments of the invention;

FIG. 13 illustrates a plot of the trajectory of the player of FIG.12 overlaid on a playing area;

FIG. 14 illustrates a user device interface (GUI) in accordance with some embodiments of the invention.

The above-mentioned drawings illustrate exemplary embodiments of the disclosed methods and systems in which like reference numerals refer to the same parts throughout the different drawings. Components in the drawings are not necessarily to scale, as emphasis is placed on clearly illustrating the principles of the inventions. Some drawings may use block or schematic diagrams and thus represent without showing details such as internal circuitry of components. Also, the embodiments shown in the figures are not to be construed as limiting the inventions but only as illustrative examples of an automated method and system according to the inventions that are illustrated herein to highlight the advantages of the inventions.

**Detailed Description**

The invention will now be described in detail with reference to the accompanying drawings. Provided are systems and methods of collecting detailed movements, orientation, patterns and attributes of an athlete/a team of athletes during live games, tagging and storing that data and identifying performance metrics within the data to help meet the above objectives.

In the following description, associated drawings, included claims, and other parts of the document, various details are set forth to provide a detailed understanding of the disclosure and embodiments thereof. It will be apparent, however, that the disclosed embodiments may be practiced without some of these details. Several features described hereafter can each be used independently of one another or in combination of other features.

Although the system can be used to simultaneously track and process data in almost any real time sporting event, such as car racing, (figure, speed) skating, (alpine, cross-country, water) skiing, (ball, ice, & field) hockey, lacrosse, it will be described in this section with regard to one exemplary embodiment for the sport of Ice hockey.

Embodiments may comprise specific types of sensors to accurately track the location over time during a live sporting event. A network of rink antennas captures the sensor data and transfers the data to a computing device that processes the data using mathematical algorithms and sports-physics models and outputs performance metrics from the given data at any given time period. These metrics can be compared against other players and teams as a whole which can be used to gain insight on the athlete's skill level and areas for improvement.

A sports-physics model provides contextual information to the computation of metrics and will vary for each particular sport. The sports-physics model uses a) the layout and boundaries of the sports area (e.g. ice rink, ski mountain, water skiing lake), b) size parameters of players (height, weight, sporting apparatus etc.), and c) connection model to relate how sensors on the athlete relate to measured positions, which then transfer to estimates of velocity/ acceleration, and then force. For example, mountain skiing can have movement in three dimensions, whereas water skiing and skating are usually in two dimensions. A bobsled may move in 3D but is constrained to a very narrow path. Skates, skis, and tires make curvilinear motion in slightly different ways, which can further be characterized by friction, sharpness of edges, and angle of lean. The sports-physics model can further include kinesiology parameters and equations to define how each muscle in the athlete's body is related to a component of acceleration. Some muscles cause the acceleration, other endure externally caused acceleration, others have no contribution or only contribute in certain directions / sporting poses.

In accordance with the present disclosure, the term "player" refers to an athlete participating in a sport such as ice hockey. Therefore, as used herein, the terms "player", "athlete", and like refer to the same interpretation and may be interchangeably used throughout the specification.

In accordance with the present disclosure, the term "strength" is used to explain the ability related to a player's power and explosiveness, allowing for more effective skating, shooting, and checking. This physical power translates directly into better execution of technical skills.

Conditioning refers to the player's ability to perform optimally throughout practice, a game or a season given the physical and mental demands of the game. Conditioning ensures that a player can maintain high levels of performance throughout the game. With superior conditioning, players can consistently apply skills under varying conditions without dropping performance.

Endurance in an athlete refers to the ability to sustain prolonged physical activity over an extended period, allowing them to perform at a high level without succumbing to fatigue. High endurance levels mean that a player can remain effective, even late in the game, which is crucial for maintaining tactical discipline and winning games. Strength, conditioning and endurance are, among others, some metrics that are calculated as part of the present disclosure to evaluate an athlete's performance during live play.

In accordance with the present disclosure, the term "performance" is described as a means to evaluate an athlete's skill/fitness level using a combination of key metrics calculated from a set of data. Therefore, the terms "performance", "skill", "skill level", "fitness", "fitness level" and the like refer to the same interpretation and may be interchangeably used throughout the specification.

"G-force", or gravitational force refers to the acceleration experienced by an athlete as a multiple of the acceleration due to Earth's gravity. In athletic contexts, g-forces can significantly impact performance, especially in sports involving rapid movements, jumps, or changes in direction. For instance, during an ice hockey game, athletes experience increased g-forces that can affect their stability, balance, and overall physical response. Understanding and adapting to these forces is crucial for athletes to enhance their performance while minimizing the risk of injury, as excessive g-forces can lead to strain on muscles and joints. Proper training helps athletes build the strength and agility needed to effectively manage and exploit g-forces during game play.

For purposes of the present disclosure, g-force is a metric/parameter calculated using sensor data to assess the performance of an athlete. Therefore, as used herein, the terms "g-force", "gravitational force", "load" and the like refer to the same interpretation and may be interchangeably used throughout the specification.

Linear acceleration as described in the present disclosure, refers to the rate of change of an athlete's velocity in a straight line, regardless of their path. It can apply to any movement, whether straight or curved.

Tangential acceleration, on the other hand, specifically refers to the rate of change of velocity along a curved path. It is concerned with changes in the speed of the athlete moving along that path, while centripetal acceleration (radial acceleration) is responsible for changing the direction of the athlete's velocity as the athlete moves along that path. Centripetal acceleration refers to the acceleration directed toward the center of a circular path. It allows an athlete to maintain a curved trajectory without moving outward.

Total acceleration is a combination of tangential and centripetal acceleration. Total acceleration, in the context of this specification can be specified as either left acceleration, which is acceleration along a curved path to the left and right acceleration which is acceleration along a curved path to the right. In a straight line the centripetal acceleration tends to zero and therefore only tangential acceleration remains. Therefore, the tangential acceleration of an object travelling in a straight line is similar to its linear acceleration.

For purposes of the present disclosure, the g-force of each type of acceleration and deceleration is calculated to assess the performance of an athlete. Therefore, as used herein, the term "left acceleration" can also refer to the term "left force" and the term "right acceleration" can also refer to the term "right force" as well the term "linear acceleration" can also refer to the term "linear force" and may be interchangeably used throughout the specification.

For purposes of the present disclosure, deceleration refers to when a player is losing speed. Deceleration can be separated into linear, left and right decelerations. Therefore, as used herein, the terms "deceleration", "deacceleration", "de-acceleration" and the like refer to the same interpretation and may be interchangeably used throughout the specification.

Furthermore "deceleration force" can also refer to individual or combined types of decelerations such as "left deceleration", "right deceleration", "linear deceleration" and the like.

In accordance with the present disclosure, the term "timestamp" is described as an instance of time. For example, a timestamp may include the instance of time each data point is recorded or measured. As used herein, the terms "timestamp", "time instance", "instance of time" and the like refer to the same interpretation and may be interchangeably used throughout the specification.

In accordance with the present disclosure, the term "timestep" is described as a period of time. For example, a timestep may include a duration of time when a certain activity (e.g. passing, shooting, skating with puck) or a game scenario occurred. As used herein, the terms "timestep", "time interval", "time period" and the like refer to the same interpretation and may be interchangeably used throughout the specification.

Embodiments further assist players in learning the best techniques, defensive plays, etc. This is done through capturing data throughout a game or from many game scenarios, identifying the common traits amongst the worst movements and best movements, presenting the benchmark to the player and identifying the players performance as compared against the benchmarks.

Embodiments can improve the accuracy of existing sports data by processing the data using mathematical algorithms and outputting metrics that give valuable insight into the performance of each individual player and the team as a whole. The measurements are categorized to improve the accuracy and transparency of sports data and to provide a better understanding of which techniques perform better than others.

The system may detect the x,y,z location of each player at any given time during a live sporting event, determine notable metrics from the data collected and identify peaks which are classified as g-force events and correspond to a physical event on ice (turns, acceleration, hits, etc.) Further classification is provided showcasing a trajectory of different accelerations at a given time or over a period. Certain key aggregates of the g-force data are categorised and presented as relating to the player's performance resulting in a database of performance metrics which provides a means to better assess a player's skill level, predict outcomes based on the measurements and improve the accuracy of existing sports data analytic sets.

The raw sensor data is refined and standardized by filling in missing data points using mathematical algorithms to have a larger set of equally-spaced data points for each player at a given timestamp. The data is converted into a refined x,y location, velocity and acceleration data set by applying a mathematical algorithm. In some embodiments, the mathematical algorithm can be a Kalman filter or a deviation thereof.

KALMAN FILTER

A Kalman filter is a mathematical algorithm used for estimating the state of a dynamic system from a series of incomplete and noisy measurements. It operates in two main steps: prediction and update.

Prediction: The filter uses the current state of the system and a mathematical model to predict the next state. It also estimates the uncertainty in this prediction.

Update: When new measurements become available, the filter updates its predictions based on these observations, taking into account the noise and uncertainty in the measurements. It combines the predicted state with the new measurements to produce a more accurate estimate.

A Kalman filter can estimate acceleration from the positions of players over time by modeling the system's dynamics and applying recursive estimation techniques. The Kalman filter processes noisy position measurements to infer the hidden variables, such as velocity and acceleration, by utilizing a state-space model that includes the relationships between position, velocity, and acceleration. To determine acceleration, the filter processes sequential measurements of player positions, estimating their velocities based on the difference in positions over time. By further differentiating the velocity, the filter can derive acceleration, allowing it to effectively smooth out noise and provide the ultimate estimation of players' motion.

A state model or a state-space model is a mathematical framework used to describe the behavior of a dynamic system by defining its current status, or state, in terms of relevant variables such as position, velocity, and acceleration. The model typically consists of two key elements: state transition model, which predicts how the system evolves over time based on its previous state, and an observation model or a measurement model, which relates the system's state to measurable outputs (e.g., sensor readings). The state model allows for the estimation of hidden variables and future states, often under conditions of uncertainty, using techniques like Kalman filters or particle filters and the like. For example, the Kalman filter can determine acceleration from position measurements by the following steps:

State-Space Model

The system's initial state can be defined to include position, velocity, and acceleration. In some embodiments including two-dimensional motion (e.g., along the x-y axes), the state vector $P$ can also be known as the initial state covariance matrix P. For example, the state vector $P$ can be written as:

$$P = \begin{bmatrix} x\ y\ v_x\ v_y\ a_x\ a_y \end{bmatrix}$$

where x and y are the positions of the player, $v_x$ and $v_y$ are the velocities and $a_x$ and $a_y$ are the accelerations in the x and y directions. Matrix $P$ defines the errors between the different parameters in the initial state estimates.

State Transition Model

The state transition model predicts the next state based on the current state. Assuming constant velocity motion, in some embodiments, the state transition matrix $Q$ can also be known as the transition state covariance matrix $Q$. For example, the state transition matrix $Q$ can be written as:

$$Q = \begin{bmatrix} 1\ \Delta_t\ 0\ 0\ 0\ 1\ 0\ 0\ 0\ 0\ 1\ \Delta_t\ 0\ 0\ 0\ 1 \end{bmatrix}$$

Where $\Delta_t$ is the time step between measurements. This matrix assumes that position changes based on velocity over time. In some embodiments, the basic form of the state update is a vector/matrix product including summation. For example, at time $k$, the state vector $x_k$ can be written as:

$$x_k = Qx_{k-1} + w_{k-1}$$

Where $Q$ is the state transition matrix, $x_{k-1}$ is the state vector at the previous time step and $w_{k-1}$ is the process noise. In some embodiments, matrix $Q$ defines the errors between the same state parameters within each time step, due to, for example, noise in the system.

Measurement Model

The measurement model defines how the actual state vector is mapped to the measurements that can be observed. It can help the filter update its state estimate based on new information received from sensors or other data sources. In some embodiments, the measurement model can be expressed as a vector/matrix product including summation. For example, at time k, the measurement vector $z_k$ can be written as:

$$z_k = Rx_k + v_k$$

Where $R$ is the measurement noise/error matrix (a.k.a. observation covariance matrix), $x_k$ is the state vector at time $k$ and $v_k$ is the measurement error at state $k$. For example, the measurement noise/error matrix can be written as:

$$R = \begin{bmatrix} 0.5^2 & 0 \\ 0 & 0.5^2 \end{bmatrix}$$

Where 0.5 represents the maximum error in the measurement device in the x and y coordinates. In some embodiments, the maximum error can be adjusted to the match that of the measurement device.

In some embodiments, the measurement model can be further expressed by a vector/matrix product including summation. For example, the next state $x_{k+1}$ can be written as:

$$x_{k+1} = Fx_k + wz_k = Hx_k + v_k$$

Where $x_k$ is the current state. In the above equation, the next state $x_{k+1}$ is modelled by the current state at $k$ moved by a process defined by $F$ and some noise $w$. Since no system follows a mathematical model perfectly, the noise $w$ is added to the equation. Similarly, since no measurement is perfect, some error $v_k$ is added.

In the above equation, $F$ is the state process which describes how the system evolves over time. For example, the state process $F$ can be written as:

$$F = \begin{bmatrix} 1 & dt & 0.5(dt)^2 & 0 & 0 & 0 \\ 0 & 1 & dt & 0 & 0 & 0 \\ 0 & 0 & 1 & 0 & 0 & 0 \\ 0 & 0 & 0 & 1 & dt & 0.5(dt)^2 \\ 0 & 0 & 0 & 0 & 1 & dt \\ 0 & 0 & 0 & 0 & 0 & 1 \end{bmatrix}$$

where $dt$ is the difference in time between state $k$ and $k + 1$. These values can be derived from existing kinematic equations.

Prediction and Update Steps

The Kalman filter operates in two phases: Prediction and Update; Prediction: Based on the previous state estimate, the Kalman filter predicts the next state (position, velocity, and acceleration) using the state transition model. Update: The filter then corrects its predictions using the observed position or measurement model and Kalman gain, which determines how much to adjust the state estimate based on the new measurement and which optimally combines the predicted state and the measurement, taking uncertainties into account.

Recursive Estimation of Acceleration

At each time step, the Kalman filter predicts the player's position, velocity, and acceleration based on the previous estimates and the state transition model and updates these predictions using the new position measurement, refining the estimate of the current acceleration.

Over time, even though only position measurements are available, the Kalman filter can accurately estimate the player's velocity and acceleration by taking advantage of the relationship between position, velocity, and acceleration in the state model.

CURVATURE AND ACCELERATION

An object such an athlete that is traveling along a trajectory may experience different types of acceleration at any given time; t. Total acceleration is a combination of tangential acceleration which is acceleration that is parallel to the direction of motion and centripetal acceleration which is acceleration that is directed towards the center of a circle that approximates the curve. In some embodiments, tangential acceleration is estimated and calculated from the Kalman filter. Total acceleration is an important parameter used to calculate forces such as g-force experienced by an athlete along a trajectory at any given time. In some embodiments, total acceleration and forces such as g-force can be calculated by applying a sports-physics model as explained below.

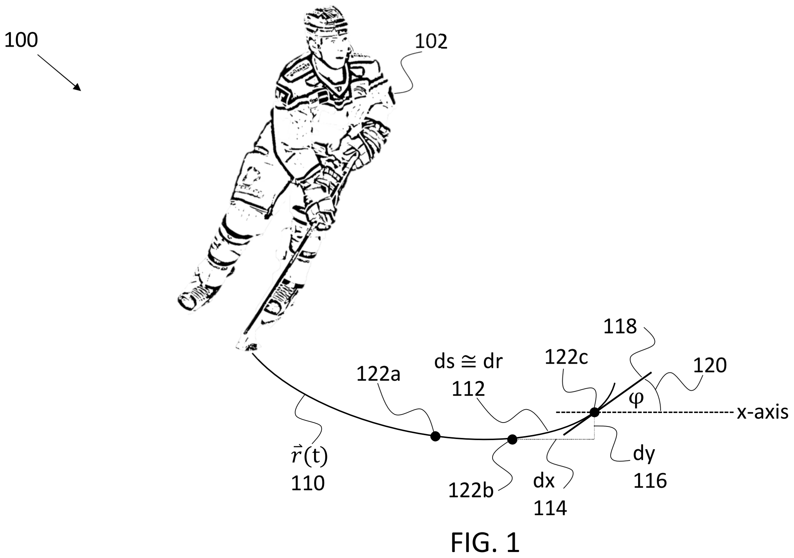

FIG. 1 shows an example segment of a trajectory **100** of a hockey player in motion at a given timeframe. As shown in FIG. 1, the player **102** is travelling along a curvilinear path **110** mathematically represented as $\vec{r}(t)$. Assuming that a timestep/time interval dt is approximately 0.1 seconds and distance **112** mathematically represented as $ds$ is the distance between point **122b** and point **122c** along the path **110** at the time interval $dt$, then $ds$ (distance **112**) can be treated as a line segment $dr$ which, in some embodiments can be approximated by Pythagorean theorem as written below:

$$ds \approx dr$$

$$dr = \sqrt{dx^2 + dy^2}$$

Where $dy$ is approximated as the vertical component **116** of $dr$ (a.k.a. rise) and $dx$ is approximated as the horizontal component **114** of $dr$ (a.k.a. run). Unit vector $\hat{T}$ points in the direction that is tangent (shown as tangent line **118**) to the curve (e.g., path **110**) at any given moment. This new vector is used to indicate the direction of travel. In some embodiments, the direction of travel can include a two-dimensional component. For example, vector $\hat{T}$ can be written as:

$$\hat{T} = \frac{d\vec{r}}{ds} = \frac{dx}{ds}\,\hat{\imath} + \frac{dy}{ds}\,\hat{\jmath}$$

Using $\hat{T}$, the velocity of player **102** travelling along path **110** can be defined. In some embodiments, the velocity calculation can include the addition of a two-dimensional component. For example, velocity $\hat{v}$ can be written as:

$$\hat{v} = \frac{d\vec{r}}{dt} = \frac{dx}{dt}\,\hat{\imath} + \frac{dy}{dt}\,\hat{\jmath}$$

Speed is defined as the magnitude of velocity. In some embodiments, the speed calculation can involve Euclidean norm/Pythagorean theorem. For example, speed $|\hat{v}|$ can be written as:

$$|\hat{v}| = \sqrt{\left(\frac{dx}{dt}\right)^2 + \left(\frac{dy}{dt}\right)^2}$$

By combining the above equations, the motion of player **102** can be adequately described as a combination of the speed and orientation of player **102**. For example, velocity $\hat{v}$ can thus be written as:

$$\hat{v} = \frac{d\vec{r}}{dt} = \frac{d\vec{r}}{ds}\frac{ds}{dt} = \frac{ds}{dt}\hat{T} = |\hat{v}|\hat{T}$$

A person of skill in the art will appreciate that slope **120** is defined as rise (e.g., *dy*) over run (e.g., *dx*). In other words, slope **120** is the angle $\varphi$ created between the x-axis and the tangent line **118** at a point such as point **122b.** Curvature, $\kappa$ of path **110** measures the rate at which the direction of the curve changes at a point along path **110**. For example, at point **122b** along path **110,** the curvature $\kappa$ can be measured as:

$$\kappa = \left|\frac{d\varphi}{ds}\right|$$

In some embodiments, radius of curvature $R$, which approximates the radius of a circle of path **110** at a point such as point **122b** can be calculated by taking the inverse of curvature $\kappa$. For example, radius of curvature $R$ can be written as:

$$R = \frac{1}{\kappa}$$

A person of skill in the art will appreciate that the vector that is perpendicular or normal to the tangent line such as tangent line **118** that is pointed to the center of the curve is typically sufficient to describe the direction of a turn along path **110**. For example, if $\hat{T}$ describes the direction of travel then $\hat{N}$ describes the direction the path **110** is turning. In some embodiments, this may imply that $\hat{N}$ is equivalent to the rate of change of the direction of travel. For example, $\hat{N}$ can be written as:

$$\hat{N} = \frac{\frac{d\hat{T}}{ds}}{\left|\frac{d\hat{T}}{ds}\right|}$$

$\hat{N}$ is therefore defined as a function of angles, for example:

$$\hat{N} = -sin\,\varphi\,\hat{\imath} + cos\,\varphi\,\hat{\jmath}$$

Using the above information, the acceleration of player **102** along a curvilinear path such as path **110** can be calculated. In some embodiments, the calculation can include a sequence of steps. For example, the acceleration $\vec{a}(t)$ can be written as:

$$\vec{a}(t) = \frac{d}{dt}(\vec{v}(t)) = \frac{d}{dt}\left(\frac{ds}{dt}\hat{T}\right) = \frac{d^2s}{dt^2}\hat{T} + \frac{ds}{dt}\frac{d}{dt}(\hat{T})$$

Where:

$$\frac{d}{dt}(\hat{T}) = \frac{d\hat{T}}{ds}\frac{ds}{dt}$$

Where:

$$\frac{d\hat{T}}{ds} = \frac{d}{ds}(cos(\varphi)\,\hat{\imath} + sin(\varphi)\,\hat{\jmath}) = -\frac{d\varphi}{ds}sin(\varphi)\,\hat{\imath} + \frac{d\varphi}{ds}cos(\varphi)\hat{\jmath}$$

$$\frac{d\hat{T}}{ds} = \frac{d\varphi}{ds}(-sin(\varphi)\,\hat{\imath} + cos(\varphi)\hat{\jmath}) = \frac{d\varphi}{ds}\hat{N}$$

Combining the above equations together, in some embodiments, the acceleration of player **102** can be defined as a function of $\hat{T}$ and $\hat{N}$. For example, the acceleration $\vec{a}(t)$ can thus be written as:

$$\vec{a}(t) = \frac{d^2s}{dt^2}\hat{T} + \left(\frac{ds}{dt}\right)^2\frac{1}{R}\hat{N} = \frac{d^2s}{dt^2}\hat{T} + \frac{|\vec{v}|^2}{R}\hat{N} = a_T\hat{T} + a_N\hat{N}$$

Where $a_N$ is the centripetal acceleration of player **102** which is defined as the acceleration that is directed towards the center of a circle that approximates the curve. Along a straight line the curvature $\kappa$ is non-existent therefore the radius of curvature $R$ is zero and this term trends to zero and there is only tangential acceleration $a_T$, that is acceleration of player **102** only parallel to the direction of motion.

Therefore, in some embodiments, the magnitude of the total acceleration of player **102** can be defined as a function of tangential acceleration and centripetal acceleration involving Pythagorean theorem. For example, the magnitude of the total acceleration $a_{total}$ can be written as:

$$a_{total} = \sqrt{a_T{}^2 + a_N{}^2}$$

The total force experienced by the player **102** is proportional to the mass of the player **102,** $m$ which can be in units of kg. Therefore, in some embodiments, the total acceleration $a_{total}$ of player **102** can include a product calculation. For example, the total force $F_{total}$ can be written as:

$$F_{total} = ma_{total}$$

Assuming that earth's gravity, $g$ is approximately 9.81m/s$^2$, the total force measurement, $F_{total}$ can be used to calculate the g-force experienced by player **102.** In some embodiments, the calculation can include fractions. For example, g-force can be written as:

$$g - force = \frac{a_{total}}{g}$$

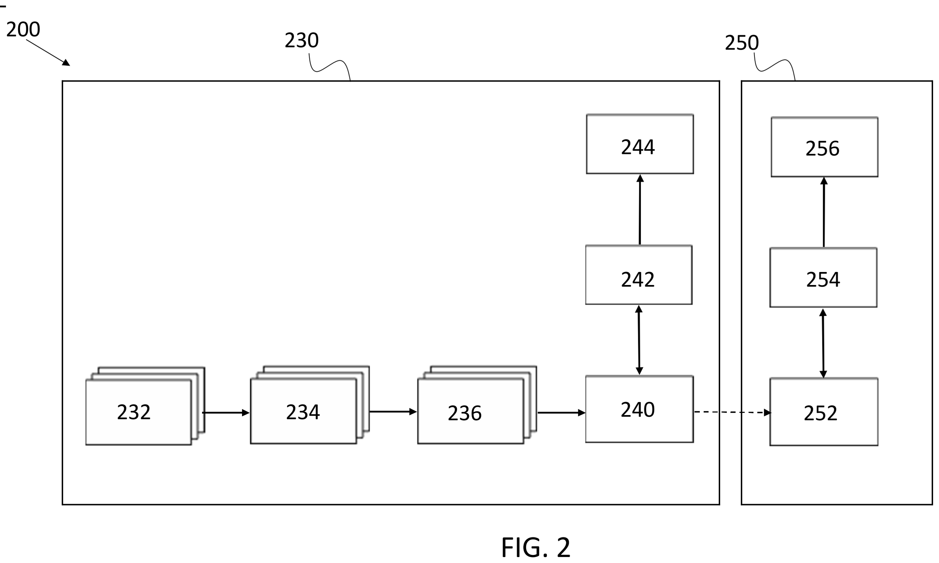

FIG. 2 illustrates a block diagram of the system's **200** components in accordance with the present disclosure. As shown in FIG. 2, a cloud computing system **250** is used to collect and consolidate data from a plurality of local systems **230**. In the preferred embodiment, the cloud computing system **250** is a centralized framework that consolidates a plurality of data from a plurality of timestamps during a single sporting event. It may comprise a cloud computing device **252,** which is used to receive and process data into a usable format, a database component **254** consisting of one or more databases and data stores to archive the data and handle data relationships, and a user interface component **256** that is able to access the data.

In some preferred embodiments of the local system **230,** a single or plurality of tracking devices such as player sensor tags **232** in use during a live sporting event communicates with wireless data transceivers **234** which are used to determine player location using multilateration which can include any standard multilateration technique, including two-way ranging, time difference of arrival, signal strength-based multilateration, phase difference of arrival or angle of arrival to name a few. Gateway devices **236** receives the location data of each player sensor tag **232** and transmits the data to edge computing device **240**. The data collected by the local edge computing device **240** is stored in a local database **242** along with a timestamp corresponding to the time the data is received. Following the tracked event, the data collected by the local edge computing device **240** is streamed to the cloud computing device **252** using an ethernet or a wireless communication network such as Wi-Fi® for post-processing.

In some embodiments, each player sensor tag **232** stores location data with timestamps. The data is offloaded to cloud computing device **252** after the tracked event for further processing, thereby minimizing data loss. However, such embodiments would require a player sensor with higher power consumption, more memory capacity and a microcontroller equipped with a clock to produce the timestamps. In some embodiments, the data is streamed directly to a cloud computing system **250** without saving in the local database **242**. Some embodiments of the local system **230** utilize a local user interface **244** to access local data or to configure aspects of the local system **230** such as identifying the location of wireless data transceivers **234**.

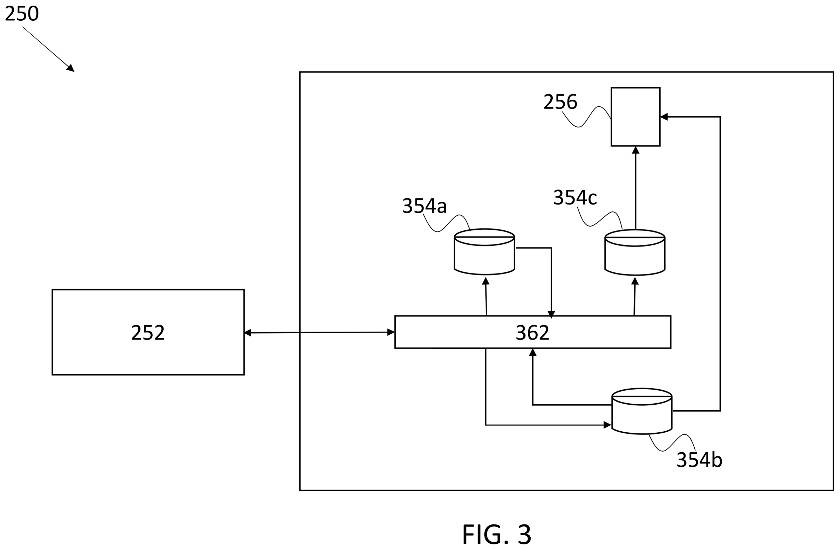

FIG. 3 illustrates an in-depth block diagram of the cloud computing system **250** of FIG. 2. Raw data received from edge computing device **240** is streamed to data pipeline **362** of cloud computing device **252** which is archived in a plurality of databases such as database **354a** and database **354b**. In some embodiments, database **354a** can be known as an "events database" which stores certain game specific events indexed by a timestamp. For example, player entered zone x at time t1, player scored a goal at time t2 and so on. These entries can come from existing data from previously tracked events as well as be added from the data pipeline. Database **354b** can also be known as a "status database" which stores all the data processed by data pipeline **362** at every interval of time such as every tenth of a second. Some of the data stored in database **354b** can include player ID, refined x and y location, speed, acceleration, g-force data as well as curvature, skating lean, etc.

In some embodiments, data pipeline **362** processes the entire dataset in stages. The corresponding quality metrics derived from g-force and other measurements can be stored in a database such as database **354c** where it can be accessed by the user interface **256** via for example a web application or an Application Programming Interface (API). User interface **256** can be, for example, a Graphical User Interface (GUI). In some embodiments, the raw data processed by data pipeline **362** is processed by a remotely accessible central computing system separate from the cloud computing system **250**. In such embodiments, the central remote system is equipped with the necessary computing resources such as memory capacity and processing power.

In some embodiments, the raw data is processed in real-time as opposed to post-processing of data as explained above. In such embodiments, the data collection, processing, storage and representation are implemented at the event site (e.g., ice rink) and are performed by the local system **230** as opposed to the cloud computing system **250**. Each player sensor tag **232** may invoke its own unique data processing pipeline to ensure smooth operation of each of the other player sensor tags **232**. For example, should one player sensor tag **232** not be in range or has a latency or dropped data, etc. the processing may still be concurrently carried out for other player sensor tags **232** without any disruptions. A further aspect of real-time processing is that the player sensor tag data is collected and processed in batches where the batch size may depend on the time interval size or the input data size as opposed to a stream of data such as the raw data stored in database **354a**.

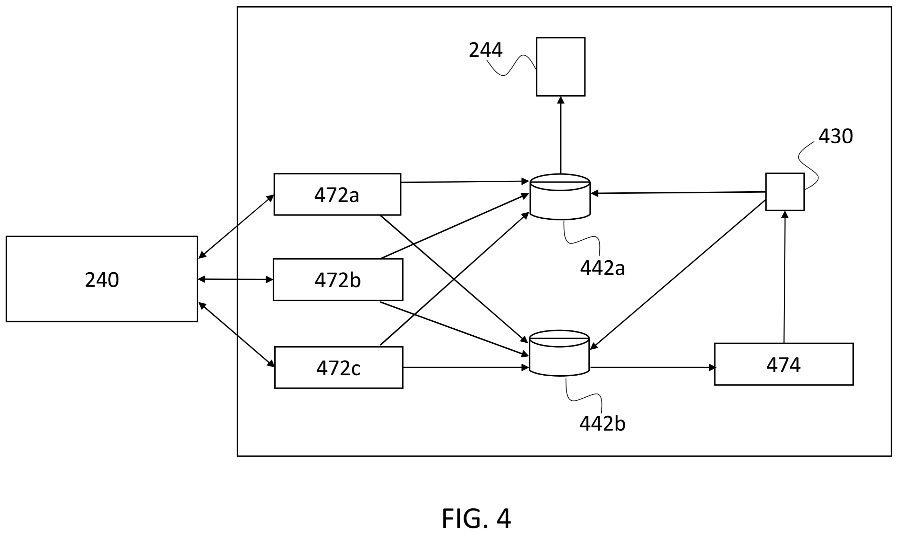

FIG. 4 illustrates an in-depth block diagram of the edge computing device **240** when it is configured to process the data in real-time. The plurality of batch data of each player is collected and processed in their own data pipeline such as the plurality of data pipelines **472** on the edge device via for example, message queuing telemetry transport (MQTT), which may concurrently process the data of each player. In some embodiments, each of the plurality of data pipelines **472** creates a time-series record in local database **442b** and updates its sensor properties (e.g., player attributes) in local database **442a**. A person of skill in the art will appreciate that the plurality of data pipelines **472** may record the batch data in the plurality of local databases **442** using write streams.

Data pipeline **474** reads the data in local database **442b** as it updates, and produces data consolidated from each individual pipeline of the plurality of data pipelines **472** in the form of team and tactical based statistics such as performance/quality metrics. The processed data **430** is stored in local database **442a** where it can be accessed by the local user interface **244** via for example a web application or an API. Local user interface can be, for example, a GUI.

In some embodiments, local database **442a** is updated at approximately every second with data received from each of the plurality of data pipelines **472**. Therefore, local database **442a** incurs an approximately one (1) second delay to receive processed data **430**. In other embodiments, local database **442a** is updated with a higher or lower time interval. A person of skill in the art will appreciate that as long as the latency of receiving processed data is within a reasonably short time interval (e.g., within 5 seconds), the data processing can be considered as real-time processing.

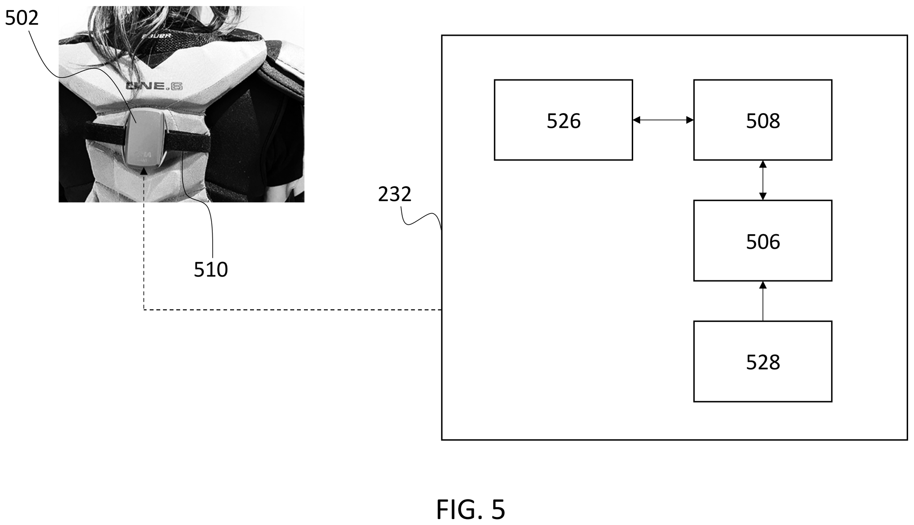

As FIG. 5 illustrates, the components of a player sensor tag **232** which may include a player sensor tag housing **502** of negligible weight. The player sensor tag housing **502** may be made of non-metal material such as plastic. Some preferred embodiments of the player sensor tag housing **502** can be secured to the player's shoulder pads using straps **510** such as elastic or nylon straps or it can be held into place with sports tape or suitable adhesives. Other embodiments can consist of the player sensor tag housing **502** that is secured directly onto the player's shoulder pads or any other suitable protective wear worn by the player via for example, a sewed in pocket. The player sensor tag **232** may be worn close to the center of mass of the player which is typically close to the spine in order to provide more accurate measurements. Some other embodiments can couple player sensor tag **232** to the athlete's stick or athlete's skate.

In some preferred embodiments, the player sensor tag **232** includes a controller **506,** wireless transceiver **508,** an antenna **526** and an on-board power supply **528.** Some preferred embodiments of the wireless transceiver **508** utilizes an ultra-wideband (UWB) chip in order to identify precise location in a sports field using multilateration to an accuracy level greater than that which typical GPS, photogrammetry, video, Bluetooth or WIFI multilateration is able to accomplish.

Antenna **526** is used to send and receive signals from the player sensor tag **232** and the wireless transceiver **508.** The signals sent between the player sensor tags **232** and the wireless data transceivers **234** are used to determine position using a multilateration algorithm. The preferred embodiment identifies location using time-of-flight signal processing from UWB transmitted signal, processing distances between the player sensor tags **232** and a plurality of wireless data transceivers **234** using a two-way ranging method to determine distances with high accuracy and a multilateration algorithm to determine location, whereas others embodiments may use different signal processing techniques to determine distances between player sensor tags **232** and wireless data transceivers **234,** for example one based on a time of flight computation such as trilateration or an angle of arrival method such as triangulation.

The controller **506** is used to pass data through the various components within the system and distribute to the wireless transceiver **508** for broadcasting through the antenna **526.** The antenna **526** may also receive broadcast information that is passed through to the wireless transceiver **508** and, in some embodiments, the controller **506.** Some embodiments may have multiple transceivers and antennas where one is used to send or receive data and the other is used to identify device location using time-of-flight signal processing methods.

Player sensor tag's **232** power supply **528** provides power and may have a plurality of power supply **528** options available, such as a rechargeable or non-rechargeable battery. The battery may be recharged by a cable attached to a charging source, such as a standard AC electrical source, universal serial bus, FireWire, ethernet or Thunderbolt wire or by inductive charging. In the preferred embodiment, the player sensor tags **232** along with wireless data transceivers **234** generate the calculated position of a player in a Cartesian coordinate system. For example, wireless data transceivers **234** transmits x,y,z coordinates corresponding to the player's location at a given time. The gateway devices **236** receive the location data via the wireless data transceiver's **234** UWB network, processes the data and transmits the data via a WI-FI network to the edge computing device **240.**

In some embodiments, a plurality of gateway devices **236** are used to stream the data from the wireless data transceivers **234** to the edge computing device **240.** Gateway devices **236** act as the intermediary between wireless data transceivers **234** and edge computing device **240** allowing communication between the two.

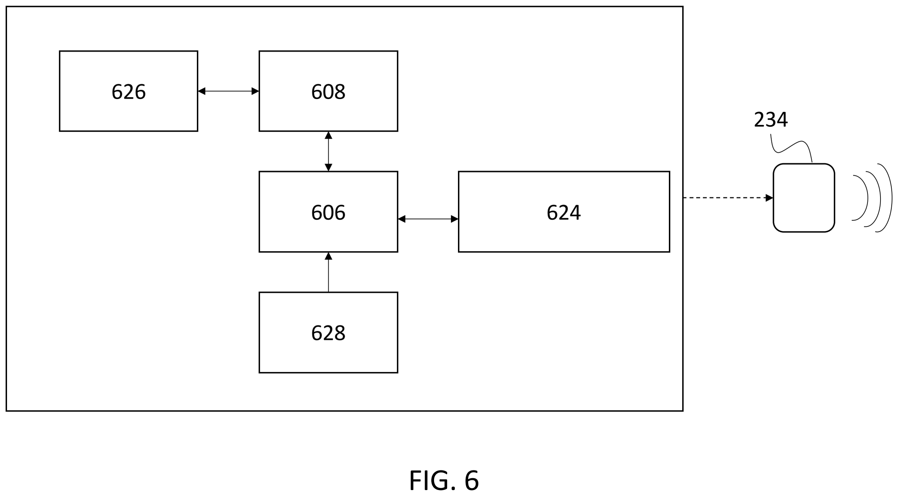

FIG. 6 is a block diagram of some preferred embodiments of the invention illustrating certain components contained within the wireless data transceivers **234.** An antenna **626** is used both to send and receive signals from the player sensor tags **232** or edge computing device **240** and send to the wireless transceiver **608.** The signals sent between the player sensor tags **232** and the wireless data receivers **234** are used to determine position using a multilateration algorithm. Some preferred embodiments identify location using time-of-flight signal processing from ultra wide-band transmitted signal, processing distances between a player sensor tag **232** and a plurality of wireless data transceivers **234** using a two-way ranging method to determine distances with high accuracy and a multilateration algorithm to determine location, whereas others embodiments may use different signal processing techniques to determine distances between player sensor tags **232** and wireless data transceivers **234,** for example one based on a time of flight computation or an angle of arrival method.

Some embodiments of the wireless data transceivers **234** may utilize a plurality of antennas such as antenna **626** and wireless transceivers such as wireless transceiver **608** for added functionality, for example to receive signals on a plurality of wireless networks, frequency channels, signal directions, or for using a variety of multilateration methods, or one to dedicate to signal processing and another for data transmission. The controller **606** within the wireless data transceiver **234** is used to pass data through the various components within the system. In some embodiments of the wireless data transceiver **234,** a network connection device **624** is used to send and receive data with an edge computing device **240** and in some embodiments also to send and receive data with other wireless data transceivers **234.** This network connection device **624** can be, for example, a serial connection, wireless data connection or an ethernet connection.

As shown in FIG. 6, each wireless data transceiver **234** is powered by power supply **628.** In some embodiments, the power supply **628** may be a battery. Power supply **628** may be built into the wireless data transceiver **234** or removable from the wireless data transceiver **234,** and may be rechargeable or non-rechargeable. In an embodiment, the power supply **628** may be in the form of a power-over-ethernet device or from a standard AC electrical connection where the wireless data transceivers **234** are only powered when connected. In an embodiment, the power supply **628** may be recharged by a cable attached to a charging source, such as a standard electrical plugin, universal serial bus, FireWire, ethernet or Thunderbolt wire. In another embodiment, the power supply **628** may be recharged by inductive charging, wherein an electromagnetic field is used to transfer energy from an inductive charger to the power supply **628** when the two are brought in close proximity, but need not be plugged into one another via a cable. In other embodiments, the wireless data transceivers **234** may be repowered by replacing power supply **628** with another power source.

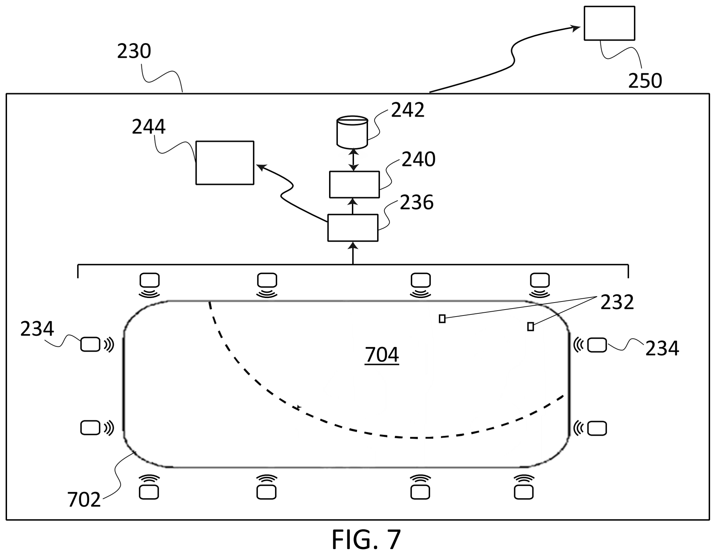

FIG. 7 illustrates an embodiment where a plurality of wireless data transceivers **234** are set up at a playing area **702.** The placement of the wireless data transceivers **234** is adjustable depending on the environment and user preferences as long as sufficient number (preferably three or more receivers, more preferably 10-12 receivers) are present and setup evenly spaced around the rink and at staggered heights to give Z-dimension depth where the lowest wireless data transceiver **234** should be near level to the height of an athlete's head and the highest wireless data transceiver **234** should be no more than the distance rating of the transceiver device such that any area contained within the entire playing area **702** has coverage from the transmitting range **704** (*i.e.,* the partial area within the dashed line of playing area **702),** of at least four wireless data transceivers **234.**

The system is configured to uniquely identify each individual player and their location in order to differentiate one player from another. For example, each player sensor tag **232** may have a unique mac address or ID in its firmware, which are transmitted.

Some preferred embodiments would identify each unique player sensor tag **232** with a particular time associated with it and the three-dimensional location relative to within the arena where the players are being tracked in. Some embodiments show the player location data as only on a two-dimensional plane.

In certain embodiments, each player sensor tag **232** is set to transmit a set of data including the player sensor tag identifier and the relative player location in x, y, z coordinates at a suitable rate. In some embodiments, the transmitting rate may be five (5) times per second when tracking two full teams of participants, and whereas in some embodiments the data transmission rate can be set to exceed five (5) times per second per player for greater measurement accuracy. In some embodiments where greater than ten (10) sets of measurement data per second are transmitted and recorded, proportionately more wireless data transceivers **234** should be used to ensure the entire system's ability to handle scaled up performance applications or a higher volume of player sensor tags **232** being tracked at higher data transmission levels.

In some embodiments, the edge computing device **240** would use proportionately additional processing power and RAM to handle the load and its disk space to store a potentially larger local database **242** than for some preferred embodiments, or if data is being streamed to a cloud computing system **250,** a proportionately higher uploading speed network connection with proportionately more stable bandwidth access could be used. Alternative to a network connection, an embodiment could store all the collected data locally and later be transferred to cloud computing system **250** for future processing or have a central remote system available for processing. In yet another embodiment, the data can be processed in real-time on site by the edge computing device **240** as previously explained.

In some preferred embodiments, the player sensor tag's **232** rate of transmission is adjustable whereby stationary players, such as spares or players that are in-waiting or in the penalty box, are set to transmit data at a lower rate than those that are in active play or near the puck. In some embodiments, an accelerometer located on the player sensor tag **232** is used to trigger an increase or decrease in the player sensor tag's **232** rate of transmitting data depending on whether the player is in slow motion, fast motion or still. In some embodiments, a command sent from the edge computing device **240** can set the rate of transmission. In some embodiments, a user can initiate a data transmission rate change using a controller.

In some preferred embodiments, a data timestamp is added when the data is stored in local database **242** whereas in another embodiment the player sensor tag **232** produces a data timestamp before being transmitted. In some other embodiments, the wireless data transceivers **234** produce a data timestamp associated with the player location data.

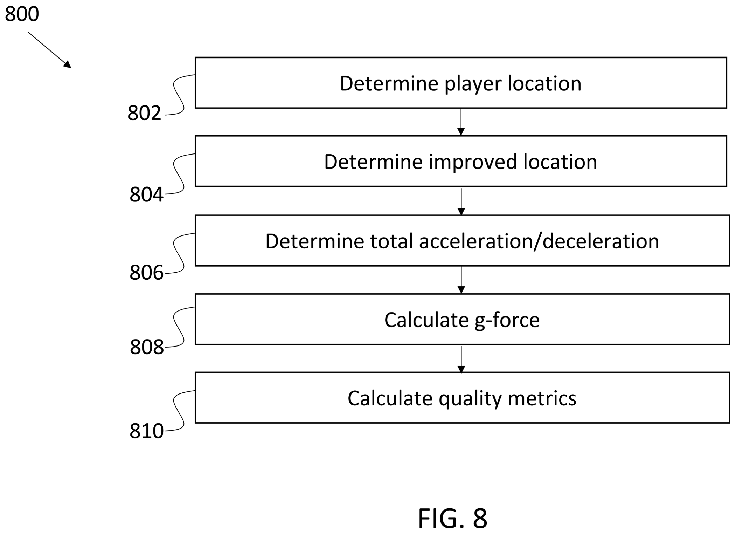

FIG. 8 outlines the process **800** to measure quality metrics that define an athlete's skill/performance in accordance with some preferred embodiments of the present invention. The first step **802** is to determine player location using player sensor tags **232**. A standard multilateration technique, including two-way ranging, time difference of arrival, signal strength-based multilateration, phase difference of arrival or angle of arrival can be applied to determine the x,y,z location of a player. In some preferred embodiments, data timestamp from each dataset is also added to local database **242** where the player sensor tag data including player location data is stored.

The second step **804** of process **800** is to determine improved location data. In an example case where a high-resolution game play event is classified, a hockey pass that takes 1.2 seconds to complete from beginning to end, measured at five (5) times per second would result in six (6) lines of data. The raw data collected may have missing data and may also not be aligned precisely to 1/5th second increments. Therefore, data cleaning steps can be taken using an algorithm such as interpolation to identify where the player location should be at any missing time based on the location data at the timestamps before and after. This same data cleaning technique can be used to increase the resolution of a player's trajectory that is captured, for example, from 1/5th second interval to 1/10th second interval, where the same or similar algorithm uses known player locations to determine where the player was located at the desired timestamp.

In some embodiments, the edge computing device **240** collects sets of transmitted data from player sensor tags **232** and transmits the raw data to cloud computing device **252** which fills in missing data points as well data points at a uniform/standardized time interval such as every tenth of a second. Cloud computing device **252** converts the new dataset including the raw data and the added data into a standardized, improved location dataset by applying a mathematical model such as a Kalman filter or a variation thereof. In some embodiments, the location data is transmitted to the cloud computing device **252** at any time during or after a game or practice. In yet another embodiment, data improvement and standardization are performed on-site by the edge computing device **240** either in real-time or following the tracked event.

The third step **806** of process **800** is to determine total acceleration and deceleration of each player. The improved location data is used by a mathematical model such as a Kalman filter and other sports-physics models to determine velocity data and to compute total acceleration and deceleration which is a combination of tangential and centripetal acceleration/deceleration. Tangential acceleration/deceleration which can be estimated and calculated from the Kalman filter is the acceleration/deceleration in the direction of motion. As previously described, centripetal acceleration/deceleration is the acceleration/deceleration in the direction of the center of a circle that estimates the curve of a player's trajectory. For purposes of the present disclosure, total acceleration/deceleration is categorized into left acceleration/deceleration, right acceleration/deceleration and linear acceleration/deceleration.

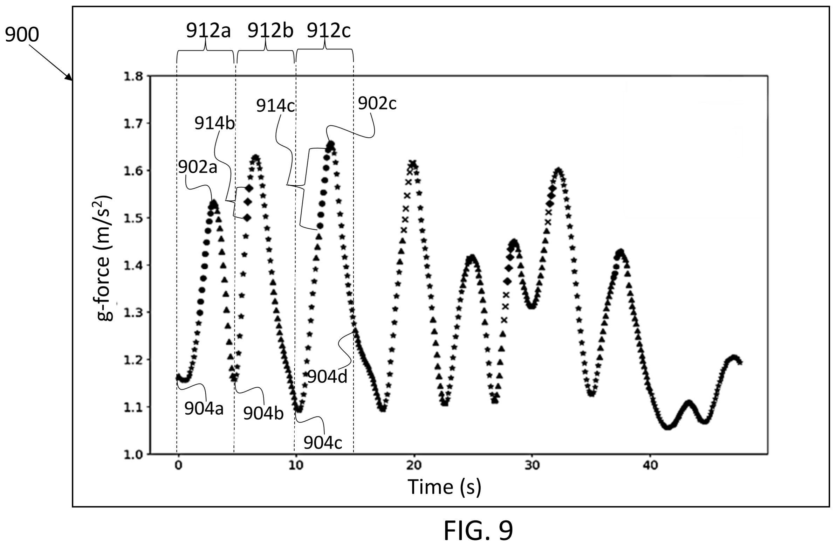

The fourth step **808** of process **800** is to calculate g-force. The calculated acceleration and deceleration data is used to define g-force, which is proportional to the total acceleration/deceleration and inversely proportional to the earth's gravitational force. By using the acceleration, deceleration and g-force data, a further set of quality metrics/measurements are calculated which can be related to a player's performance/fitness using the process in FIG. 8 and further described in FIG. 9, FIG. 10, FIG. 11, FIG. 12 and FIG. 13.

The fifth step **810** of process **800** is to apply mathematical formulae to the g-force values to calculate quality metrics that ultimately evaluates an athlete's performance/fitness. These metrics are also known as performance metrics which give tactical insight and statistics to evaluate players and teams.

FIG. 9 illustrates a plot **900** of g-force versus time of a player during a certain time interval of a tracked event. Each peak of FIG. 9 such as peak **902c** is classified as a g-force event which corresponds to a physical event on ice (e.g., turns, acceleration, hits, etc.) Different g-force events can be categorized and given an effort classification as shown in Table 1 below:

Table 1

| G-force | Effort Classification |
| --- | --- |
| 1-1.25 | Min |
| 1.25-1.5 | Low |
| 1.5-1.75 | Medium/Moderate |
| 1.75-2 | High |
| 2-2.5 | Overexertion |
| 2.5+ | Hard stop/Strong hit/Outlier |

In the above table, players are associated with effort classifications based on g-force. Further classification can be provided as to what type of g-force event occurred at the given timestamps such as left/right acceleration and deceleration and linear acceleration and deceleration and so on. A g-force peak above 2.5 is an experimental value that has been realized through testing and is considered to be found in very hard stops along boards and/or very strong hits where acceleration changes instantaneously.

As previously described, an aspect of the present disclosure is to determine the type of acceleration a player is using for any necessary action during practice/gameplay such as turns, stops and starts, etc. In order to determine the type of play such as left/right acceleration or deceleration as shown in FIG. 9, FIG. 10, FIG. 11, FIG. 12 and FIG. 13, one needs to determine the direction of the player and/or skating angle. In some embodiments, the sports-physics model can be used to calculate an athlete's direction of motion and/or skating angle. For example, using three sequential positional data points obtained from the player sensors such as player sensor tag **232,** the angle of the player's trajectory (i.e., skating angle) can be calculated to determine whether the player has turned to the left or right. For example, by taking the x,y coordinates of three consecutive points A, B and C at approximately 0.1(s) intervals of player **102** of FIG. 1 where point A corresponds to point **122a,** point B corresponds to point **122b** and point C corresponds to point **122c,** the angle theta ($\theta$) between the vectors AB (from A to B) and BC (from B to C) formed at point B can be calculated. In some embodiments the calculation can use an inverse trigonometric formula with an inner product. For example, the formula can be written as:

$$\theta = arccos \left( \frac{AB \cdot BC}{|AB| \cdot |BC|} \right)$$

The resulting angle quantifies the player's change in direction. In some embodiments, a skating angle in the range of -5 degrees to 5 degrees indicates that player **102** travelled linearly (i.e., in a straight line). Consequently, a skating angle outside the range of -5 degrees to 5 degrees is considered to be angular.

A further calculation can be used to determine if player **102** turned to the left or right. In some embodiments a two-dimensional cross product formula (or determinant) can be used. For example, the formula can be written as:

$$Cross\ Product = (x_2 - x_1)(y_3 - y_2) - (y_2 - y_1)(x_3 - x_2)$$

Where $x_1$ and $y_1$ are the x,y coordinates of point A, $x_2$ and $y_2$ are the x,y coordinates of point B and $x_3$ and $y_3$ are the x,y coordinates of point C. A positive result indicates a left turn (counterclockwise), while a negative result indicates a right turn (clockwise).

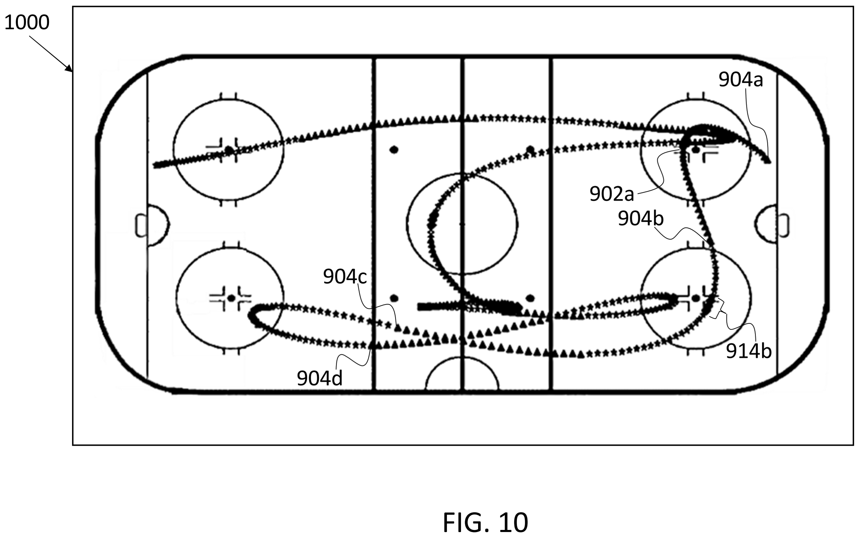

FIG. 10 illustrates the g-force vs. time graph of FIG. 9 represented as a trajectory **1000** on a playing area such as playing area **702** of FIG.7. As shown in FIG. 9 and FIG. 10, the triangles represent linear acceleration, the dots represent left acceleration, the crosses represent right acceleration, the stars represent linear deceleration, the squares represent left deceleration and the diamonds represent right deceleration.

As can be seen by window **912a** of FIG. 9 the player is accelerating from rest (e.g., point **904a)** and makes a left turn. At the cusp of the peak **902a** the player makes a transition from left acceleration to linear acceleration, which indicates that the player is gaining speed out of their first turn. During the time period of window **912b** (e.g., from point **904b** to point **904c),** the player skates linearly but with a reduced acceleration and makes a right turn while decreasing their speed, which is indicated by the right deceleration markers (*i.e.* diamonds) shown in window **914b.** Furthermore, the player has made a wide right turn at window **914b** as can be seen in FIG. 10. Out of the turn the player continues to decrease their speed for a short time then picks up speed and skates linearly towards their next turn.

During the time period of window **912c** (e.g., from point **904c** to point **904d),** the player begins linearly decelerating, applies a short amount of linear acceleration as they go into a sharp left turn as shown in FIG. 10 at which point the player records the highest g-force of the entire time period as can be seen by peak **902c.** As they exit the turn they begin to linearly decelerate. As the player progresses towards the next turn past point **904d,** they linearly accelerate as indicated by the triangle markers of FIG. 9 and FIG. 10 immediately following point **904d.**

As previously mentioned, various quality metrics can be derived from the g-force data. For example, force output, force capacity, active load, exertion during play, recovery and so on. In some preferred embodiments of the present invention, force output is also known as strength which relates to the highest g-force experienced by the player during a tracked event. For example, peak **902c** of FIG. 9 is the highest g-force recorded by the player and it is therefore considered the maximum strength of that player. In a game scenario, the force output or strength can be related to acceleration bursts experienced by the players during sprint or puck pursuit acceleration as well as high speed tight turns and edges.

In some preferred embodiments, force capacity is also known as conditioning and can be related to the force output. For example, force capacity can be written as:

$$force\ capacity = \frac{force\ output}{average\ force\ output} \times 100$$

Where average force output can be the mean or median average of the peak g-force values. Average force output can be calculated by adding the sum of all peak g-force values and dividing by the number of peaks/g-force events. In some embodiments, force capacity or conditioning is monitored in segments during a tracked event (e.g., fifteen (15) second segments). Conditioning refers to the player's ability to perform optimally throughout practice, a game or a season given the physical and mental demands of the game.

In the preferred embodiment, active load is also known as endurance and is related to the total of all force outputs in the tracked event. Is it calculated by adding the peak g-force values together during a tracked event. Active load or endurance is a subset of conditioning. It is a measure of a player's ability to sustain energy and performance levels over time, whether through repeated high-intensity shifts or throughout an entire game.

In some preferred embodiments, exertion at work or during gameplay is a measure of the percentage of time a player experienced g-force higher than a set value. In some embodiments the set value may be 1.25. Exertion refers to the amount of effort a player puts into a specific activity at any given time. Fatigue is measured as the percentage of time the player is exerting less than the set value (e.g., g-force of 1.25). Therefore, fatigue is inversely proportional to exertion.

At rest or recovery is another important metric in some preferred embodiments. It measures the percentage of time a player is not fully engaged in play. For example, during shift changes as well as at work when the player exerts minimum or low-effort as shown in Table 1. This measure gives insight into which players should be playing and which players are in need of rest during a gameplay.

The g-force values can also be used for injury prevention. For example, the system may flag anomalies such as a force output of a player that is higher than the typical/average force output of the same player which indicates that the player has exerted a higher-than-normal g-force. Such a scenario can be analyzed further to determine whether the player has improved their performance and if not, it could be an indicator of potential injury. Furthermore, a player that is returning from an injury can be evaluated against their benchmark quality metrics to determine whether or not the player is ready to return to play.

In the preferred embodiment, g-force is categorized into the type of acceleration or deceleration for example, linear, right and left acceleration and deceleration and each quality metric described above is measured against the different types of acceleration and deceleration. For example, FIG. 9 can be dissected up to six more figures where each figure shows the g-force data of the player when they were accelerating linearly, accelerating to the right, decelerating to the left and so on.

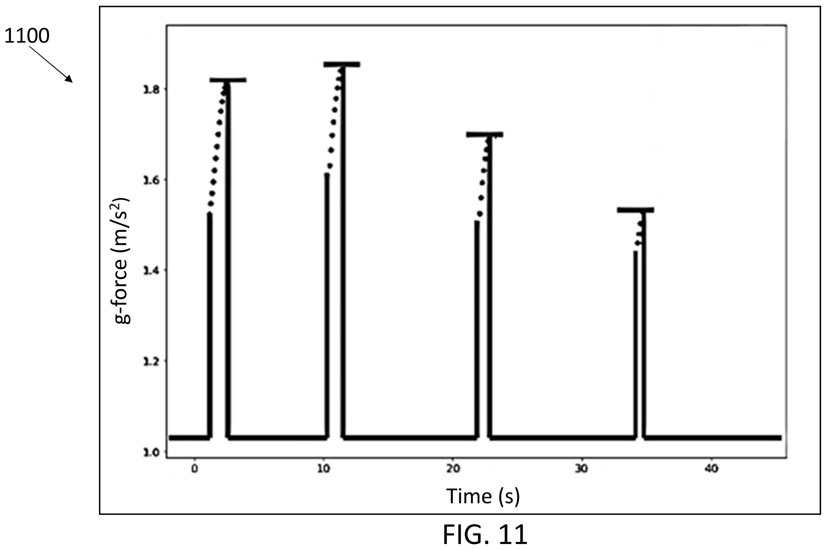

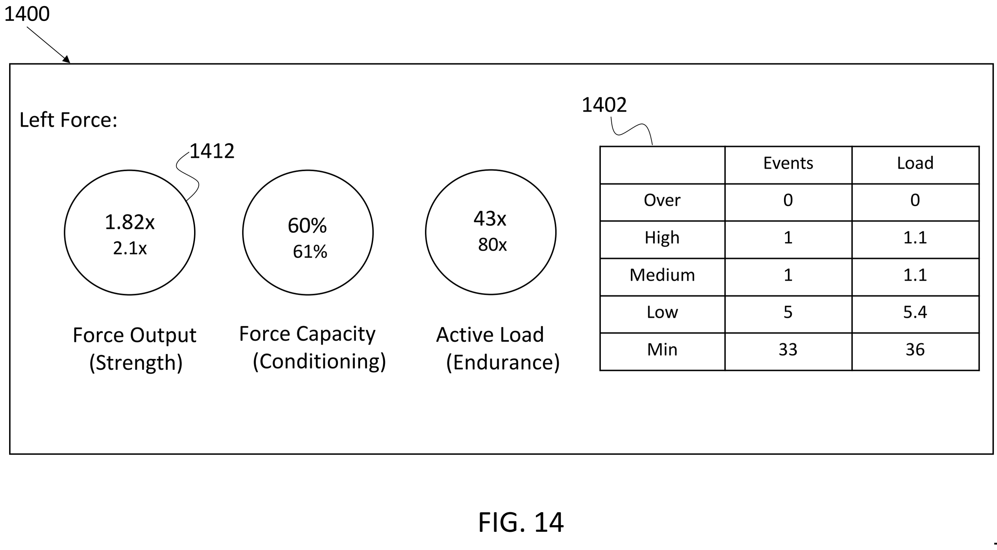

FIG. 11 illustrates a plot **1100** of g-force versus time of a player different from the player of FIG. 9 but FIG. 11 shows the data for left acceleration only. Using the data of FIG. 11, the processor calculates different quality metrics such as force output, force capacity and so on categorized under left acceleration. As shown in FIG. 14, quality metrics of left acceleration are shown as "Left force". Using the separate g-force data for each type of acceleration and deceleration, quality metrics such as force capacity can be calculated for each different type of acceleration or deceleration. This allows for in-depth analysis of each player and identifies their strengths as well areas that need improvement.

Another object of some preferred embodiments of the present disclosure is to estimate a player's lean angle, also known as "lean" when accelerating around a curve. The lean angle may therefore act as another quality metric when evaluating a player's performance/skill with regards to skating quality and technique. Lean can be described as function of radius of curvature *R* and the speed of the athlete such as player **102** of FIG. 1. In some embodiments, the lean calculation can include an inverse trigonometric formula. For example, lean calculation can be written as:

$$Lean = tan^{-1}(\frac{(R \times g)}{|\hat{v}|^2})$$

Where *g* is the acceleration due to the earth's gravitational force typically measured as $9.81 m/s^2$ and $|\hat{v}|$ is the speed of the player **102** at a point such as point **122c** along a path such as path **110.** Speed is the Euclidean sum of the velocities in the x and y direction at point **122c.**

The lean measurements can also be used to measure g-force per muscle group since the lean angle can indicate how the player's skates are being used (e.g., outside edge, inside edge) as well as which dominant muscle groups are involved. Therefore, in some embodiments, the sports-physics model includes kinesiology parameters and equations to define how each muscle or the dominant muscles in the athlete's body is related to a component of acceleration.

Lean angle characterizes the direction of a turn, for example, leaning to the right would cause the player to turn to the right. Therefore, determining the quality metrics of a right turn may also characterize the dominant muscle groups involved in that turn (e.g. gluteus maximus and medius, quads and calf muscles of the left leg, core obliques, and adductor muscles of the right leg) which gives valuable insight on the player's skating ability. Determining the quality metrics for the type of acceleration or deceleration at each turn can therefore be used for coaching feedback on how to improve skating performance for example, by applying more/less weight on the back/front foot as well as which leg is more dominant and which leg needs to be stronger.

Another advantage of angle of skate and g-force per muscle group measurements is to Compare equipment quality such as skates when used by the players. For example, the equipment quality can be tested by measuring signs of wear such as carvings on skis, profiles of ice hockey skates of one make of a set equipment when utilized by a player during a game or practice and measuring the same of another make of a set of equipment worn by the same player during another game or practice.

In some embodiments, the skate profile, sharpness or dullness can be evaluated by comparing the different types of acceleration of a player (for example, right acceleration/deceleration, left acceleration/deceleration) towards the beginning of a game and towards the end of the game or practice or between two or more games. For example, if a player recorded higher g-force values when making a right turn during a previous game than the same player making a similar right turn at a game following the previous game where the two games occurred within a short time distance (e.g., a day or a few days apart) that would indicate that either the player is fatigued or that the player's skates need sharpening or both. Since the quality metrics such as conditioning, exertion and fatigue can also indicate fatigue levels in players, by process of elimination, the degradation of player's speed between the previous game and the most recent game can give insight as to whether the player is fatigued and/or it is a matter of equipment quality.

Another outcome of determining the g-force and other quality metrics over a period of time or throughout the gameplay is for comparisons, both among multiple players and for a single player over time, to output statistical comparisons. The processor may compare one player against their cohort (e.g., gender, age, team, league, or country) to evaluate one player's performance against others. The various quality metrics described above are used for the performance comparisons.

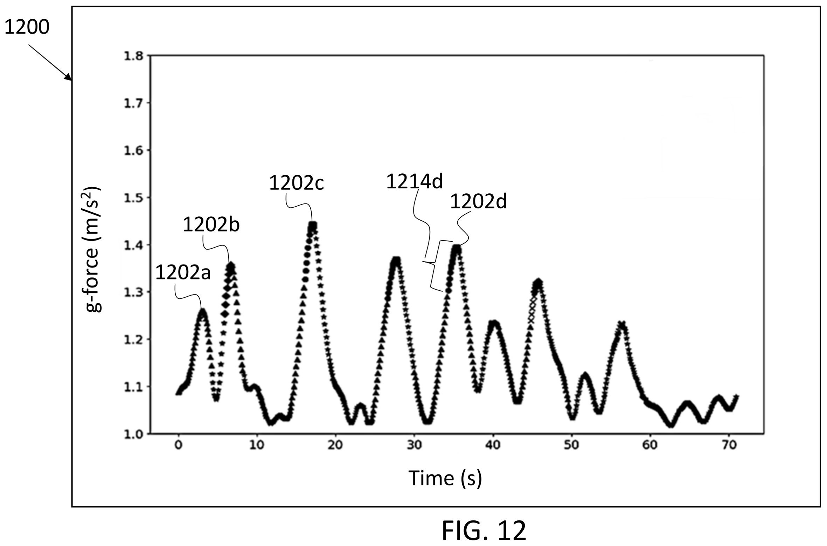

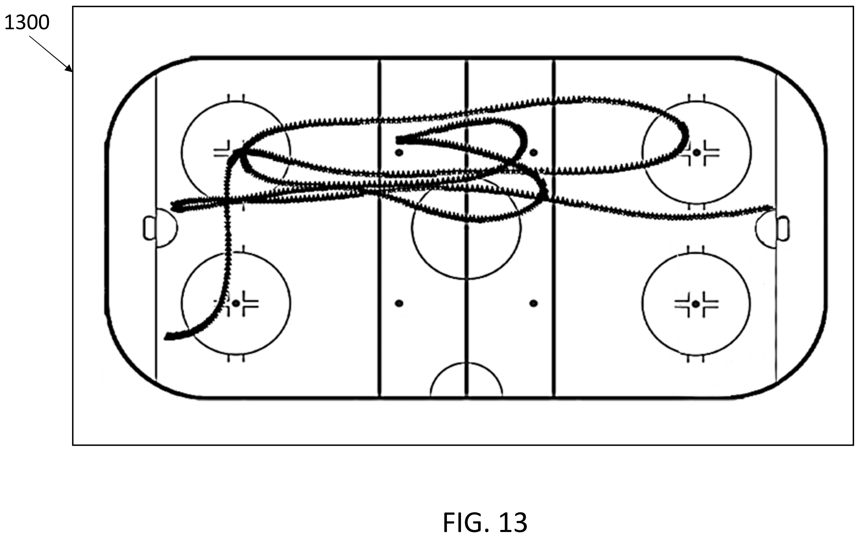

FIG. 12 illustrates a plot **1200** of g-force vs. time of another player (different from the player of FIG. 9 and FIG. 11) during a certain time interval and FIG. 13 illustrates the same data represented as a trajectory **1300** where the data is overlaid on a game area such as an ice rink. Similar to FIG. 9 and FIG. 10, in FIG. 12 and FIG. 13, the triangles represent linear acceleration, the dots represent left acceleration, the crosses represent right acceleration, the stars represent linear deceleration, the squares represent left deceleration and the diamonds represent right deceleration.

When comparing FIG. 9 and FIG. 12, it can be seen that the player of FIG. 9 showcases higher strength capacity since the max peak of a g-force event (e.g., peak **902c**) is greater than any peak of the player of FIG. 12 (e.g., plurality of peaks **1202).** Specifically, it can be seen in FIG. 9 that peak **902c** which is a left acceleration into a linear deceleration outperforms (in terms of force output) any peak of FIG. 12; however, it can be seen from the number of peaks of FIG. 9 that the player of FIG. 9 was able to sustain more g-force events above 1.25 totalling eight peaks during a 0 to 40 second interval, compared to FIG. 12's six (6) peaks. This displays a higher endurance level for the player of FIG. 9 because they sustain multiple active load events during the same time.

From FIG. 9 and FIG. 12 it is acceptable to assume that both players favor linear acceleration. To identify which type is more beneficial one must compare with the trajectory data (e.g., FIG. 10 and FIG. 13) to find the ideal path. Furthermore, the player of FIG. 12 tends to slow down as they approach a turn as can be seen by the left and right decelerations near a number of peaks. For example, during window **1214d,** the player of FIG. 12 decelerates to the left whereas the player of FIG. 9 tends to accelerate when approaching a turn as can be seen by window **914c** of FIG. 9 where the player accelerates to the left. This indicates that the player of FIG. 9 performs better during turns and that the player of FIG. 12 needs to improve their skills when approaching a turn.

Similarly, a trend may be evaluated for any given player for their own statistics over time, in a single sporting event or over the season. The processor may perform trend analysis and time-series analysis, as known in the art, to compute statistics such as: a baseline quality metric for identified events, rate of improvement, or plateaus. The processor may also evaluate changes in that player's performance such as fatigue over time for an event. For example, the system may output how a player's right turns have sped up over time or display changes of skill level over time.

Therefore, it is possible to quantify an athlete's skill level by comparing a single athlete's single or plural performance from their identified data against known quality/performance metrics of the same event type. The processor may also compute the differential between a top-level player or a set of top-level players performance data and the athlete's actual performance data to determine a skill level. The difference may be a weighted factor and/ or Root-Mean-Square of differences in quality metrics (force output, conditioning, etc.) Alternatively, the skill level is calculated by comparison of the athlete's performance data during game situations versus during practice and/or game.

In some embodiments, the processor may also compute one or more quality metrics for an identified event, by comparing the observed quality metrics to an existing database of quality metrics. The processor may compute differences in various types of play such as right, left and linear acceleration and deceleration for comparable times. In some embodiments, the processor may also compute the differences in quality metrics for each different type of play (e.g., shot, deke, puck pursuit, etc.)

The skilled person will appreciate that there can be several different ways to display the quality metrics. For example, by quantifying the data such as g-force and other quality metrics in terms of frequency of occurrence throughout a recorded event or across multiple events to output a histogram of player performance data and by comparing each player's performance against themselves as well as other players. Alternatively, the processor may compare the observed performance data to positive and negative exemplars of performance data stored in a database such as database **254** to output a comparison figure such as a histogram outlining for example, the gradient of a player's performance throughout the event or over time, the percentile of a player's performance when compared to the exemplary set, etc.

FIG. 14 illustrates an example GUI output **1400** that can be accessed by the user via either local user interface **244** or user interface **256** depending on whether the data was processed in real-time or following a tracked event. GUI output **1400** may display quality metrics such as force output, force capacity and active load broken down to the different types of acceleration or deceleration where FIG. 14 shows the quality metrics of left acceleration which is equivalent acceleration to the left. The GUI output **1400** may also show the player's quality metrics compared to the team's average. For example, icon **1412** shows the force output of a player when accelerating to the left and the team's average force output of the same type of acceleration is shown directly below. In some embodiments, the quality metrics shown in GUI output **1400** are calculated from a single tracked event. In other embodiments, the GUI output **1400** can show similar quality metrics calculated across multiple events.

Several other statistics such as the number of g-force events recorded by the player at each different type of acceleration can also be shown by the GUI output **1400**. For example, table **1402** shows the number of times the player recorded high, over high, medium, low and min g-force events (shown in the "Events" column), when accelerating to the left. The high, medium, low and min g-force events are the effort classifications as previously shown in Table 1 which indicates the amount of g-force experienced by the player.

In some embodiments, GUI **1400** may employ other types of data representation in addition to the ones described above. For example, GUI **1400** may display a plurality of charts such as pie charts, doughnut charts, clock charts with a plurality of clock arms, bar charts, gauge charts, circle graphs, etc. to display the performance metrics of a player as well as player comparison data.

Embodiments of the present disclosure provide an expert analysis of player performance that is otherwise difficult or nearly impossible to achieve through the naked eye. Even a hired coach or other existing technology, such as video replay, alone are not able to calculate these outputs at sufficient rates. The present disclosure uses mathematical models to determine acceleration data from player positions and transform the accelerations into g-force measurements, which are further processed to calculate aggregated fitness, player-to-player comparisons, strength, fatigue, skate lean and other sports IQ with respect to each individual player and the team as a whole, total g-force event counts, etc.

In some embodiments, player position can be measured by distributing one or more video cameras in or around a sports area such as playing area **702,** connecting a stream of images of an athlete/player using the one or more video cameras and using image processing and video data processing such as You Only Look Once (YOLO) segmentation to identify the athlete and their location within the images to determine a series of location data of the athlete within the sports area for a corresponding series of timestamps.

A person of skill in the art will appreciate that the present disclosure can be used in combination with other player tracking and analytic systems. For example, in some embodiments, player position can be measured by player sensor tags **232** in combination with puck/stick sensors, rink coordinate data or video data processing and player recognition.

Each document cited herein was cited to provide clarity to the reader and is incorporated by reference in its entirety. In cases where the present disclosure conflicts with a document incorporated by reference, the present disclosure controls.

While the disclosure has been described in connection with specific embodiments, it is to be understood that the disclosure is not limited to these embodiments and that alterations, modifications, and variations of these embodiments may be carried out by the skilled person without departing from the scope of the disclosure. It is furthermore contemplated that any part of any aspect or embodiment discussed in this specification can be implemented or combined with any part of any other aspect or embodiment discussed in this specification.

The above description illustrates various embodiments of the present disclosure along with examples of how aspects of particular embodiments may be implemented. The above examples should not be deemed to be the only embodiments or implementations, and are presented to illustrate the flexibility and advantages of the particular embodiments as defined by the following claims. Based on the above disclosure and the following claims, other arrangements, embodiments, implementations and equivalents may be employed without departing from the scope of the present disclosure as defined by the claims.

**Claims**

1. A method of estimating an athlete's fitness in a sporting event comprising:

   distributing plural transceivers in a sports area;
   connecting a tracking device to the athlete;
   communicatively coupling the transceivers and tracking device;
   using multilateration at the tracking device to determine a series of location data (Xi, Yi) of the athlete for a corresponding series of timestamps (Ti);
   applying a Kalman filter to refine the series of location data, and calculate a series of velocity data and series of acceleration data for the athlete;
   applying a sports-physics model to the data to calculate total acceleration at each timestamp;
   calculating and outputting forces experienced by the athlete based on the total acceleration.

2. The method of claim 1, wherein refining the series of location data further comprises interpolating the series of location data to correspond to a standard set of times.

3. The method of claim 1, further comprising calculating quality metrics and muscle loading from the outputted force, wherein the quality metrics includes one or more of: force output, force capacity, active load, endurance, exertion during play and recovery.

4. The method of claim 1, wherein calculating total acceleration includes calculating acceleration components, including tangential and centripetal acceleration of the athlete.

5. The method of claim 4, further comprising calculating skate lean or skate technique of the athlete from the calculated acceleration components.

6. The method of claim 1, further comprising displaying a trace of the athlete using the location data, overlaid on an image of the sports area, with coding to indicate a magnitude or classification of the acceleration.

7. The method of claim 1, wherein the sports-physics model is particular to a given sport, selected from one of: car racing, skating, skiing, hockey, and lacrosse.

8. The method of claim 1, a) wherein the calculated total accelerations are stored for plural athletes in the sporting event and further comprising computing comparison statistics for total acceleration between athletes or between teams, or b) wherein the calculated total accelerations are stored for plural sporting events and wherein improvement statistics are computed for the athlete between sporting events.

9. The method of claim 1, further comprising classifying acceleration components into left acceleration/deceleration, right acceleration/deceleration and linear acceleration/deceleration.

10. The method of claim 1, wherein said calculations are performed over a window of the timestamps that correspond to tracked events stored in a database for that sporting event.

11. A method of estimating fitness in a sporting event comprising:

distributing one or more video cameras in a sports area;
connecting a stream of images of an athlete using the one or more video cameras;
using image processing to identify the athlete and their location within the images to determine a series of location data $(X_i, Y_i)$ of the athlete within the sports area for a corresponding series of timestamps $(T_i)$;
applying a Kalman filter to refine the series of location data, and calculate a series of velocity data and series of acceleration data for the athlete;
applying a sports-physics model to the data to calculate total acceleration at each timestamp;
calculating and outputting forces experienced by the athlete based on the total acceleration.

12. A system for estimating fitness in a sporting event comprising:

plural transceivers distributed in a sports area;
a tracking device connectable to an athlete and communicatively couplable to the plural transceivers; and
a computer processor, memory and instructions arranged to:

use multilateration at the tracking device to determine a series of location data $(X_i, Y_i)$ of the athlete for a corresponding series of timestamps $(T_i)$;
apply a Kalman filter to refine the series of location data, and calculate a series of velocity data and series of acceleration data for the athlete;
apply a sports-physics model to the data to calculate total acceleration at each timestamp; and
calculate and output forces experienced by the athlete based on the total acceleration.

13. The system of claim 12, further comprising a display for showing a trace of the athlete using the location data, overlaid on an image of the sports area, with coding to indicate a magnitude or classification of the acceleration.

14. The system of claim 12, further comprising a data store storing the calculated accelerations for plural athletes in the sporting event and wherein accelerations comparison statistics are computed between athletes or between teams, or calculated for the athlete between sporting events.

15. The system of claim 12, wherein the tracking device is an UWB package located at one or more of: the athlete's torso, the athlete's stick, athlete's skate, or within a ball or puck.

FIG. 1

FIG. 2

EP 4 768 090 A1

FIG. 3

EP 4 768 090 A1

FIG. 4

EP 4 768 090 A1

FIG. 5

EP 4 768 090 A1

FIG. 6

EP 4 768 090 A1

FIG. 7

800

```
                  ┌─────────────────────────────────────────┐
            802 ──┤        Determine player location          │
                  └─────────────────────────────────────────┘
                                    │
                                    ▼
                  ┌─────────────────────────────────────────┐
            804 ──┤        Determine improved location        │
                  └─────────────────────────────────────────┘
                                    │
                                    ▼
                  ┌─────────────────────────────────────────┐
            806 ──┤   Determine total acceleration/deceleration │
                  └─────────────────────────────────────────┘
                                    │
                                    ▼
                  ┌─────────────────────────────────────────┐
            808 ──┤            Calculate g-force              │
                  └─────────────────────────────────────────┘
                                    │
                                    ▼
                  ┌─────────────────────────────────────────┐
            810 ──┤        Calculate quality metrics          │
                  └─────────────────────────────────────────┘
```

FIG. 8

FIG. 9

FIG. 10

FIG. 11

FIG. 12

FIG. 13

1300

1400

Left Force:

1412

| | Force Output (Strength) | Force Capacity (Conditioning) | Active Load (Endurance) |
|---|---|---|---|
| | 1.82x  2.1x | 60%  61% | 43x  80x |

1402

| | Events | Load |
|---|---|---|
| Over | 0 | 0 |
| High | 1 | 1.1 |
| Medium | 1 | 1.1 |
| Low | 5 | 5.4 |
| Min | 33 | 36 |

EP 4 768 090 A1

FIG. 14

| Distribute plural transponders in a sports area |
| --- |

| Connect a tracking device to an athlete |
| --- |

| Communicatively coupling the transponders and tracking device |
| --- |

| Use multi-lateration at the tracking device to determine a series of location data (Xi, Yi) of the athlete for a corresponding series of timestamps (Ti) |
| --- |

| Apply a Kalman filter to refine the series of location data, and calculate a series of velocity data and series of acceleration data for the athlete |
| --- |

| Apply a sports-physics model to the data to calculate acceleration at each timestamp |
| --- |

| Calculate and outputting forces experienced by the athlete based on the acceleration. |
| --- |

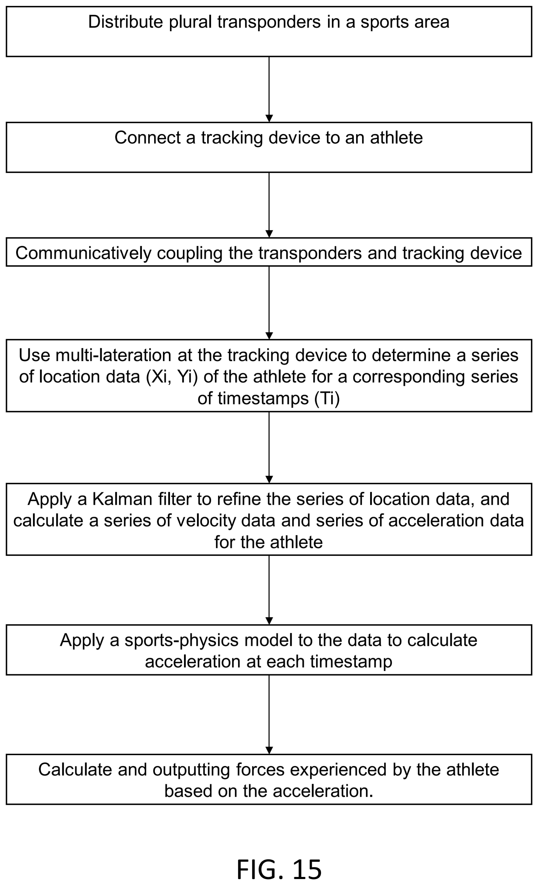

## FIG. 15

Europäisches
Patentamt
European
Patent Office
Office européen
des brevets

# EUROPEAN SEARCH REPORT

**Application Number**

EP 25 22 7746

## DOCUMENTS CONSIDERED TO BE RELEVANT

| Category | Citation of document with indication, where appropriate, of relevant passages | Relevant to claim | CLASSIFICATION OF THE APPLICATION (IPC) |
|---|---|---|---|
| X | US 6 710 713 B1 (RUSSO GAETANO TOM [US]) 23 March 2004 (2004-03-23) * abstract * * column 3, line 39 - line 50 * * column 4, line 45 - line 48 * * column 3, line 65 - line 67 * * column 7, line 3 - line 7 * * column 11, line 66 - column 12, line 3 * ----- | 1-15 | INV. A63B24/00 |
| X | US 2015/375083 A1 (STELFOX JILL [US] ET AL) 31 December 2015 (2015-12-31) * abstract * * figures 1,2a * * paragraph [0052] - paragraph [0064] * * paragraph [0099] * * paragraph [0147] * * paragraph [0179] * * paragraph [0263] * ----- | 1-15 | |
| A | US 6 204 813 B1 (WADELL BRIAN C [US] ET AL) 20 March 2001 (2001-03-20) * abstract * * column 11, line 36 - line 42 * ----- | 1-15 | TECHNICAL FIELDS SEARCHED (IPC) A63B |
| A | US 2009/231198 A1 (WALSH PETER M [US] ET AL) 17 September 2009 (2009-09-17) * abstract * * paragraph [0032] - paragraph [0034] * ----- | 1-15 | |

The present search report has been drawn up for all claims

| Place of search | Date of completion of the search | Examiner |
|---|---|---|
| The Hague | 18 May 2026 | Dhondt, Erik |

EPO FORM 1503 03.82 (P4C01)

**EP 4 768 090 A1**

## ANNEX TO THE EUROPEAN SEARCH REPORT
## ON EUROPEAN PATENT APPLICATION NO.

EP 25 22 7746

18-05-2026

| Patent document cited in search report | | Publication date | Patent family member(s) | | Publication date |
|---|---|---|---|---|---|
| US 6710713 | B1 | 23-03-2004 | NONE | | |
| US 2015375083 | A1 | 31-12-2015 | US 2015375083 A1 | | 31-12-2015 |
| | | | US 2019293431 A1 | | 26-09-2019 |
| US 6204813 | B1 | 20-03-2001 | NONE | | |
| US 2009231198 | A1 | 17-09-2009 | NONE | | |

EPO FORM P0459

**REFERENCES CITED IN THE DESCRIPTION**

*This list of references cited by the applicant is for the reader's convenience only. It does not form part of the European patent document. Even though great care has been taken in compiling the references, errors or omissions cannot be excluded and the EPO disclaims all liability in this regard.*

**Patent documents cited in the description**

- US 11179600 B
- US 20240123289 A1
- US 20240306943 A1